**Europäisches Patentamt**

**European Patent Office**

**Office européen des brevets**

(19)

(11) Veröffentlichungsnummer: **0 125 566**
**A1**

(12) ## EUROPÄISCHE PATENTANMELDUNG

(21) Anmeldenummer: **84104988.5**

(22) Anmeldetag: **03.05.84**

(51) Int. Cl.³: **C 07 C 1/24**
C 07 C 11/02, C 07 C 13/20

(30) Priorität: **11.05.83 DE 3317165**

(43) Veröffentlichungstag der Anmeldung:
**21.11.84 Patentblatt 84/47**

(84) Benannte Vertragsstaaten:
**AT BE DE FR GB IT NL**

(71) Anmelder: **BASF Aktiengesellschaft
Carl-Bosch-Strasse 38
D-6700 Ludwigshafen(DE)**

(72) Erfinder: **Dockner, Toni, Dr.
Grossgasse 6
D-6701 Meckenheim(DE)**

(72) Erfinder: **Krug, Herbert
Mussbacher Strasse 49
D-6700 Ludwigshagen(DE)**

(54) Verfahren zur Herstellung von Olefinen durch Wasserabspaltung aus Alkoholen.

(57) Herstellung von Olefinen aus Alkoholen durch Wasserabspaltung in einem hochsiedenden Mineralöl, wobei das mit Nebenprodukten angereicherte Mineralöl abgezogen und einer Verfeuerungsanlage zugeführt wird.

EP 0 125 566 A1

Verfahren zur Herstellung von Olefinen durch Wasserabspaltung aus Alkoholen

Die Erfindung betrifft ein Verfahren zur Herstellung von Olefinen aus Alkoholen durch Dehydratisierung in flüssiger Phase in einem hochsiedenden Mineralöl, wobei das Mineralöl erneuert und das mit Nebenprodukten angereicherte Mineralöl der Verfeuerung zugeführt wird.

In Gegenwart starker Säuren läßt sich aus Alkoholen in flüssiger Phase häufig sehr glatt Wasser abspalten. Dazu sind hohe Konzentrationen an starker Säure wie Schwefelsäure oder Phosphorsäure erforderlich, um ausreichende Reaktionsgeschwindigkeiten zu erzielen. Die Dehydratisierungsreaktion verläuft in der Regel bei Temperaturen von 180 bis 200°C in flüssiger Phase. Infolge dieser drastischen Bedingungen entstehen erhebliche Mengen an Nebenprodukten, z.B. durch Veretherung, Isomerisierung und Polymerisation des gebildeten Olefins.

Als der bessere Weg, um zu Olefinen zu gelangen, gilt die Dehydratisierung von Alkoholen in der Gasphase an heterogenen Katalysatoren (Houben-Weyl: Methoden der org. Chemie, Bd. 4/2, S. 209). Als Katalysator finden z.B. Aluminiumoxid, Aluminiumphosphat, Oxide des Thoriums, Wolframs und Titans oder auch Mischoxide Verwendung. Die Reaktionstemperaturen liegen mit 300 bis 450°C so hoch, daß als Nebenreaktionen vor allen Dingen Nachreaktionen und Kohlenstoffbildung eintreten können. Die Katalysatoren müssen dann mit Luft regeneriert werden. Ein weiterer Nachteil bei Verwendung von Feststoff-Katalysatoren sind die Kosten, die bei der Herstellung als auch beim Ein- und Ausbau sowie durch die Entsorgung entstehen. Schwierigkeiten während der Synthese bringt bei Gasphasenreaktionen derungünstige Stoff- und Wärmetransport, so daß größere Wärmetauschflächen und auch Überhitzung in Kauf genommen werden müssen.

Es bestand daher die Aufgabe ein Verfahren vorzuschlagen, das die genannten Nachteile nicht aufweist und das es in einfacher Weise gestattet, die Nebenprodukte abzutrennen.

Diese Aufgabe wurde mit einem Verfahren zur Herstellung von Olefinen durch Dehydratisierung von Alkoholen in flüssiger Phase erfindungsgemäß dadurch gelöst, daß man die Dehydratisierung durch Einleiten des Alkohols in flüssigem oder gasförmigem Zustand in ein hochsiedendes Mineralöl bei Temperaturen oberhalb des Siedepunkts des Alkohols und des sich bildenden Olefins durchführt, das Olefin gasförmig abzieht, das hochsiedende Mineralöl bei Anreicherung mit Nebenprodukten erneuert und das mit Neben-Hp/Kl produkten angereicherte hochsiedende Mineralöl abzieht und vorzugsweise einer Verfeuerung zuführt.

Unter hochsiedenden Mineralölen werden hochsiedende Raffinerieprodukte verstanden mit einem Siedepunkt 150°C, wie Gasöl, Vakuumgasöl, Heizöl S, technisches Weißöl, geschmolzenes Paraffinwachs oder aromatisches Kohlenwasserstofföl. Vorteilhaft wird Vakuumgasöl mit einem Siedepunkt von über 200°C, insbesondere mit einem Siedebereich zwischen 350°C und 500°C verwendet.

Als zu dehydratisierende Alkohole kommen beliebige zur Wasserabspaltung befähigte Alkohole, die unter den Reaktionsbedingungen beständige Olefine bilden, insbesondere aliphatische, cycloaliphatische oder araliphatische Alkohole ohne weitere funktionelle Gruppen in Betracht.

Als aliphatische Alkohole sind solche mit 2 bis 20 Kohlenstoffatomen, beispielsweise Ethanol, Butanol oder Decanol zu nennen.

Zur Wasserabspaltung befähigte cycloaliphatische Alkohole sind ein- und mehrkernige Verbindungen, beispielsweise Cyclohexanol, Cyclododecanol, 1,2,3,4-Tetrahydronaphthol-1 oder 1,2,3,4-Tetrahydronaphthol-2.

Als araliphatische Alkohole kommen beispielsweise 2-Phenylethanol oder 4-Phenylbutanol-1 in Betracht.

Die Dehydratisierung kann je nach Art der Reaktion ohne oder vorzugsweise mit Katalysatoren durchgeführt werden. Bei Verwendung von Katalysatoren können sowohl im Mineralöl unlösliche als auch im Mineralöl lösliche Katalysatoren verwendet werden, die demgemäß im Mineralöl gelöst, emulgiert oder suspendiert sind.

Bevorzugt verwendet man saure Katalysatoren wie aliphatische oder aromatische Sulfonsäuren, z.B. Benzolsulfonsäure oder Toluolsulfonsäure, Dodecyclbenzolsulfonsäure, Schwefelsäure und Schwefelsäurehalbester wie Alkylschwefelsäure, Phosphorsäure oder deren partiell veresterte Derivate oder Borsäure und deren saure Derivate. Man kann auch Säureanhydride wie Phosphorpentoxid, Schwefeldioxid und Boroxid verwenden.

Die im Mineralöl löslichen Katalysatoren werden dem Mineralöl im allgemeinen in Mengen von 0,01 bis 25 Gew.%, vorzugsweise 0,1 bis 10 Gew.%, insbesondere 0,5 bis 5 Gew.%, bezogen auf das Mineralöl, zugesetzt.

Als suspendierte Katalysatoren kommen Aluminiumoxid, Aluminiumphosphat, Borphosphat, Aluminiumsilikat, Kieselgel, Titanoxid, Heteropolysäuren des Phosphors und Molybdän- und Wolframsäure in Betracht.

Die Dehydratisierungsreaktion wird im allgemeinen bei Temperaturen von 50°C bis 600°C, vorzugsweise 50 bis 550°C, insbesondere 150 bis 350°C, durchgeführt. Im allgemeinen werden Atmosphärendruck oder erhöhter Druck angewendet. Es ist jedoch auch möglich, bei vermindertem Druck zu arbeiten.

Als Reaktoren sind für die Dehydratisierungsreaktion z.B. Rührkessel geeignet. Vorteilhaft werden jedoch für das neue Verfahren senkrecht angeordnete zylindrische Reaktoren wie Glockenbodenkolonnen, Blasensäulen oder Füllkörperkolonnen verwendet. Der Ausgangsstoff oder die Ausgangsstoffe werden in der Regel gasförmig am Boden des mit Mineralöl gefüllten Reaktors zugeführt bzw. flüssig am Boden des Reaktors zugeführt, wobei bei flüssiger Zuführung das Mineralöl bei einer Temperatur gehalten wird, die höher ist als die Siedetemperatur der flüssig zugeführten Ausgangsstoffe. Es kann vorteilhaft sein, den verdampften Ausgangsstoff mit einem inerten Gas zu verdünnen. Geeignete inerte Gase sind z.B. Wasserdampf, Kohlendioxid und vorzugsweise Stickstoff.

Die Reaktionsprodukte werden gasförmig am Kopf des Reaktors abgezogen. Anschließend werden die gasförmigen Reaktionsprodukte zweckmäßig kondensiert. An die Kondensation kann noch eine Reinigungsstufe, z.B. eine Destillation oder Fraktionierung angeschlossen werden.

Das neue Verfahren kann diskontinuierlich oder kontinuierlich durchgeführt werden, wobei jedoch die kontinuierliche Arbeitsweise bevorzugt wird. Bei der kontinuierlichen Arbeitsweise kann es vorteilhaft sein, das Mineralöl kontinuierlich zuzuführen und abzuziehen, beispielsweise wenn es sich um eine Reaktion handelt, bei der Crackprodukte gebildet werden. Diese Crackprodukte werden dabei mit dem Mineralöl kontinuierlich aus dem Reaktor ausgeschleust. Eine Aufarbeitung und Rückführung des abgezogenen Mineralöls ist in der Regel nicht wirtschaftlich, da das Mineralöl, z.B. als Heizöl oder Vakuumgasöl in der Regel wohlfeil zur Verfügung steht. Man wird daher zweckmäßig das abgezogene Crackprodukt enthaltende Mineralöl einer Verfeuerung un dem Reaktor frisches Mineralöl zuführen.

Das neue Verfahren hat gegenüber demjenigen des Standes der Technik wesentliche Vorteile:

Man kommt mit katalytischen Mengen Säure aus oder kann auch rein thermisch spalten, und die Reaktionstemperatur liegt im allgemeinen 50 bis 100°C tiefer als bei der Dehydratisierung an heterogenen Katalysatoren in der Gasphase. Polymere, Crack- und höhersiedende Nebenprodukten verbleiben im Mineralöl, das nicht regeneriert zu werden braucht und das, gegebenenfalls nach Abtrennen des Katalysators, zweckmäßig dem Kraftwerk zugeführt wird. Bei der Dehydratisierung im "Ölbett" finden keine oder keine nennenswerten Isomerisierungsreaktionen statt und die Ausbeuten liegen höher. Ferner ist das Verfahren in der technischen Ausführung wesentlich wirtschaftlicher als Gasphasenreaktionen an heterogenen Katalysatoren, wie sie in der Literatur zahlreich beschrieben sind und auch in der Praxis ausgeübt werden. Schließlich bewirkt die Verbrennung des Reaktionsmediums und der darin enthaltenen Neben- und Crackprodukte eine verringerte Umweltbelastung.

## Beispiel 1

Die Apparatur besteht aus einem elektrisch beheizten Quarzrohr (D = 60 mm, L = 1100 mm), das mit 2,6 Liter Glasringen (D = 5 mm), 1,4 kg Vakuumgasöl (Kp 400°C) und 5 Gew.% Dodecylbenzolsulfonsäure gefüllt ist. Über ein Tauchrohr werden bei 205°C stündlich 74,1 g tert.-Butanol mittels einer Dosierpumpe zugeführt. Gleichzeitig leitet man 2,5 l/h Stickstoff ein. Das den Reaktor verlassende Reaktionsgemisch wird bei −40°C kondensiert. Dabei fallen 72,53 g Kondensat an. Dieses besteht aus 9,7 Gew.% tert.-Butanol, 22,5 Gew.% $H_2O$ und 67,8 Gew.% Isobuten. Dies entspricht einem Umsatz von 90,5 %. Die Ausbeute an Isobuten, bezogen auf umgesetztes t-Butanol, beträgt 97 % der Theorie.

## Beispiel 2

Man verfährt wie in Beispiel 1 beschrieben, hält aber die Reaktionstemperatur bei 165°C. Man erhält 72,7 g Kondensat, das 16,3 Gew.% t.-Butanol, 20,8 Gew.% $H_2O$ und 62,9 Gew.% Isobuten enthält. Dies entspricht einem Umsatz von 84 %, die Ausbeute beträgt 97 % der Theorie.

## Beispiel 3

In der in Beispiel 1 beschriebenen Apparatur speist man stündlich 100 g Cyclohexanol mit 2,5 l Stickstoff bei 215°C ein. Nach Kondensation erhält man 99,4 g eines Produktes, das aus 7,4 Gew.% Cyclohexanol, 1,5 Gew.% Cyclohexanon, 17,0 Gew.% $H_2O$ und 74,4 Gew.% Cyclohexan bestehen. Dies entspricht einem Umsatz von 93 % und einer Ausbeute von 96 % der Theorie.

## Beispiel 4

In der in Beispiel 1 beschriebenen Apparatur werden stündlich 51 Gewichtsteile Butanol-1 in 1200 Gewichtsteile Vakuumgasöl mit 60 Gewichtsteilen Dodecylbenzolsulfonsäure bei 250°C eingeleitet. Man erhält stündlich 50 Gewichtsteile Kondensat aus 44,8 Gewichtsteilen Butanol-1, 13,8 Gewichtsteilen $H_2O$, 36,2 Gewichtsteilen Buten-1, 2,8 Gewichtsteilen trans-2-Buten und 2,4 Gewichtsteilen cis-2-Buten. Dies entspricht einem Umsatz von 56 % und einer Ausbeute von 86 % Buten-1, 7 % trans-2-Buten und 6 % cis-2-Buten, bezogen auf umgesetztes Butanol-1.

0125566

### Patentansprüche

1. Verfahren zur Herstellung von Olefinen durch Wasserabspaltung aus Alkoholen in flüssiger Phase, dadurch gekennzeichnet, daß man die Dehydratisierung durch Einleiten des Alkohols in flüssigem oder gasförmigem Zustand in ein hochsiedendes Mineralöl bei Temperaturen oberhalb des Siedepunkts des Alkohols und des sich bildenden Olefins durchführt, das Olefin gasförmig abzieht, das hochsiedende Mineralöl bei Anreicherung mit Nebenprodukten erneuert und das mit Nebenprodukten angereicherte hochsiedende Mineralöl abzieht.

2. Verfahren gemäß Anspruch 1, dadurch gekennzeichnet, daß man die mit Nebenprodukten angereicherten Mineralöle einer Verfeuerung zuführt.

3. Verfahren gemäß Anspruch 1, dadurch gekennzeichnet, daß man als hochsiedendes Mineralöl Gasöl, Vakuumgasöl, schweres Heizöl, technisches Weißöl oder Vakuumrückstand verwendet.

4. Verfahren gemäß Anspruch 1, dadurch gekennzeichnet, daß man die Dehydratisierung an einem an sich für Dehydratisierungsreaktionen bekannten Katalysator durchführt.

5. Verfahren gemäß Anspruch 1, dadurch gekennzeichnet, daß man die Dehydratisierung kontinuierlich durchführt.

6. Verfahren gemäß Anspruch 1, dadurch gekennzeichnet, daß man die Dehydratisierung bei Temperaturen von 150 bis 350$^{\circ}$C durchführt.

Europäisches
Patentamt

**EUROPÄISCHER RECHERCHENBERICHT**

Nummer der Anmeldung

| | **EINSCHLÄGIGE DOKUMENTE** | | EP 84104988.5 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
| X | GB - A - 584 601 (H.G. CRUISHANK FAIRWEATHER)<br><br>* Seite 1, Zeile 92 - Seite 2, Zeile 9; Seite 2, Zeile 32; Seite 3, Zeilen 84-87,103-105; Beispiel III *<br><br>-- | 1,3-6 | C 07 C 1/24<br>C 07 C 11/02<br>C 07 C 13/20 |
| X | US - A - 2 374 405 (BALCAR)<br><br>* Seite 1, linke Spalte, Zeilen 25-27; Seite 1, linke Spalte, Zeile 55 - Seite 1, rechte Spalte, Zeile 7; Ansprüche *<br><br>-- | 1,3-6 | |
| X | US - A - 2 371 634 (LORCH)<br><br>* Seite 1, linke Spalte, Zeilen 25-27; Seite 1, rechte Spalte, Zeilen 1-9; Seite 2, rechte Spalte, Zeilen 23-46 *<br><br>-- | 1,3-6 | |
| A | DE - A1 - 3 134 107 (ANIC)<br><br>* Beispiel 6; Anspruch 1 *<br><br>-- | 1,4 | **RECHERCHIERTE SACHGEBIETE (Int. Cl. ³)**<br><br>C 07 C 1/00<br>C 07 C 11/00<br>C 07 C 13/00 |
| A | DE - B - 1 065 836 (INSTITUT FRANCAIS DU PETROLE)<br><br>* Spalte 1, Zeile 5 - Spalte 2, Zeile 51; Ansprüche 1,6 *<br><br>-- | 1,4-6 | |
| A | GB - A - 1 358 188 (BASF)<br><br>* Beispiel, insbesondere Seite 3, Zeilen 59-62; Anspruch 2 *<br><br>---- | 1,4,6 | |

Der vorliegende Recherchenbericht wurde für alle Patentansprüche erstellt.

| Recherchenort | Abschlußdatum der Recherche | Prüfer |
|---|---|---|
| WIEN | 10-08-1984 | KÖRBER |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03 82